# EUROPEAN PATENT APPLICATION

(11) **EP 3 747 812 A1**
(43) Date of publication of application: **09.12.2020**
(21) Application number: 19178698.7
(22) Date of filing: 06.06.2019
(51) Int. Cl.: B65H 63/06, G01N 21/89, G01N 33/36

(54) **SENSOR DEVICE FOR SENSING THE QUALITY OF AN ELONGATE TEXTILE BODY**

(71) Applicant: Gebrüder Loepfe AG, 8623 Wetzikon (CH)
(72) Inventor: Heusser, Gianni Willi, 8340 Hinwil (CH); Occhi, Lorenzo, 8330 Pfäffikon (CH); Wieland, Guido, 7206 Igis (CH); Zehnder, Roger Pierre, 8048 Zürich (CH)
(74) Representative: Sutter, Kurt

(57) **Abstract**

The optical sensor device comprises a slit (10) having a measurement region (6) for receiving a yarn to be measured. The lateral walls (16b, 16c) are formed, at least at the measurement region (6), by an integral body (18). In a direction along the depth of the slit (10), the distance of a top edge (24) of the integral body (18) to the center (30) of the measurement region (6) is large enough to prevent most fibers caught at the edge (24) to be kept away from the measurement region (10). This increases the accuracy of the measurement.

## Description

### Technical Field

The invention relates to an optical sensor device for sensing at least one quality parameter of an elongate textile body, such as a yarn. This type of sensor is e.g. used in quality control and monitoring for detecting contaminants in the elongate body, such as foreign fibers or vegetable matter, or for measuring the thickness of such a body. The invention also relates to a method for manufacturing such a sensor.

### Background Art

US 5371584 and EP 1508797 describe optical sensor devices for measuring foreign material in a yarn and/or the diameter of the yarn. Such devices comprise a measurement slit extending along the propagation direction of the yarn and forming a measurement region.

This type of device is sensitive to contaminations in the measurement slit.

### Disclosure of the Invention

The problem to be solved by the present invention is to provide a sensor device that is less sensitive to contamination.

This problem is solved by the device of claim 1. Accordingly, the invention relates to a sensor device for sensing at least one quality parameter of an elongate textile body, such as the body's diameter or hairiness or the presence of contaminants in the body. The device comprises at least the following elements:
- A housing.
- A measurement region for the elongate textile body: This region receives the elongate body during examination.
- A least one light source emitting light into said measurement region and at least one light detector receiving light from said measurement region: These components carry out the optical examination of the elongate textile body.
- A measurement slit extending along a propagation direction of the yarn: The measurement region is located in this slit. The slit is open at a first and second side transversal to the propagation direction. These are the sides where the yarn enters and exits the slit during examination. The measurement slit is also open at a third side extending along the propagation direction. This side extends, e.g., parallel to the yarn and can be used for inserting the yarn prior to the examination. The slit has two lateral walls and a bottom wall extending along said propagation direction. In this context, the "third side" of the slit is opposite to the bottom wall. Further, the direction perpendicular to the propagation direction and connecting the bottom wall and the third side defines a "depth direction".
- An integral body having a slit-facing surface forming at least part of each lateral wall and the bottom wall of the slit. An "integral body" is a body made of a single piece, in particular a piece formed in a single casting process. Advantageously, the integral body used herein is a single component body or a two-component cast body.

According to the invention, the distance, measured along said depth direction, between the center of the measurement region and the edge of the integral body at the lateral walls is at least 13 mm.

In this context, the "center" of the measurement region is the time-average of the elongate body's position during the measurement. It may e.g. be defined by guides associated with the sensor device.

This design is based on the understanding that the edge of the integral body forms a location where fibers (e.g. fine strands of cotton) can remain clamped at the interface between the integral body and the housing. For instance, the typical length of cotton fibers in ring spinning can be up to 25 or 35 mm. Investigations have shown that they are typically aligned roughly along the propagation direction. Hence, by locating the edge sufficiently far away from the center of the measurement region, the risk of such fibers extending into the measurement region can be reduced.

Advantageously, the housing of the device forms part of each lateral wall of the slit, i.e. both the integral body and the housing form parts of each lateral wall. Hence, the housing protects the slit at its third side mechanically and/or optically.

Advantageously, the housing and the edge of the integral body meet at a seam.

The invention also relates to a method for manufacturing such a sensor comprising the step of casting the integral body.

The sensor device is particularly suited for measuring foreign material in the elongate textile body or the diameter of the elongate textile body. In one advantageous application, the sensor device is used in a yarn clearer.

Other advantageous embodiments are listed in the dependent claims as well as in the description below.

### Brief Description of the Drawings

The invention will be better understood and objects other than those set forth above will become apparent from the following detailed description thereof. Such description refers to the annexed drawing, wherein
Fig. 1 shows a sectional 3D view of the measurement device (with only part of the housing shown) and
Fig. 2 is a sectional view along line II-II of Fig. 1.

### Modes for Carrying Out the Invention

### Definition

Terms such as "top", "bottom", "above" and "below" are used in reference to an orientation where the bottom wall is the bottommost part of the measuring slit.

The lateral walls of the slit are considered to be "smooth" at the location of the seam if there is no step at that location and if the surfaces of the lateral walls right above and below the seam have the same direction.

*Transparent* is understood to designate a *body* that is substantially transparent to the light of the light source in the sense that it absorbs or scatters no more than 20%, in particular no more than 10% of the light from the light source passing through it. A transparent *surface* is understood to designate a surface that is substantially transparent to the light of the light source in the sense that it reflects or scatters no more than 20%, in particular no more than 10% of the light from the light source passing through it.

### Overview

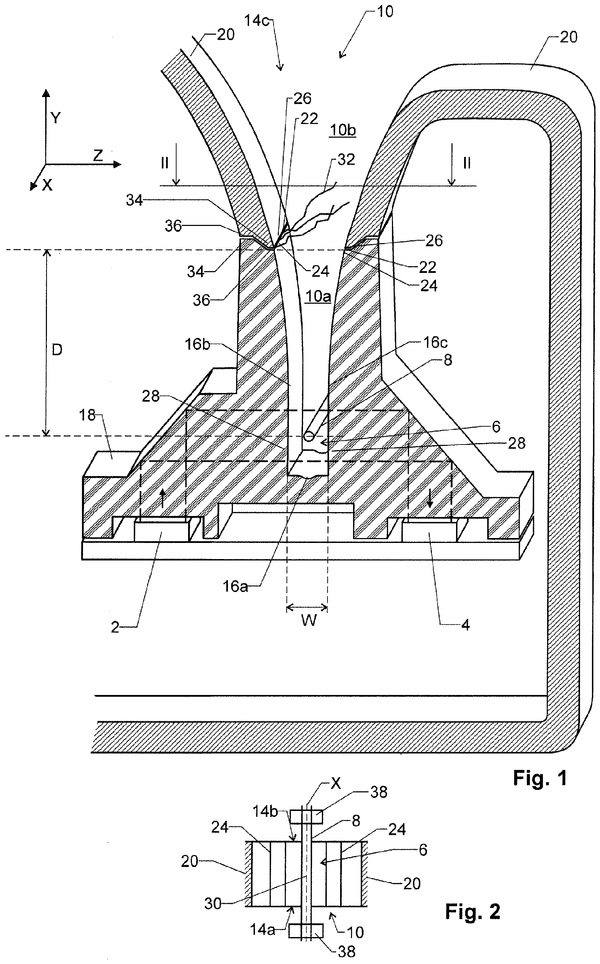

The device shown in Fig. 1 comprises a light source 2 and a light detector 4. On its way from light source 2 to light detector 4, the light passes a measurement region 6, where it interacts with elongate body 8, which may e.g. be a yarn.

The signal at light detector 4 depends on the optical properties of body 8. In the example shown here, where the measurement is carried out in transmission, it typically depends on the diameter of body 8. As known to the skilled person, when the measurement is carried out in reflection, the signal will also depend on other parameters of body 8, such as the presence of contaminants with optical properties different from the body per se.

Measurement region 6 designates the region where the body 8 is located. Since, in operation, the body's position is not stationary but fluctuates, the width and height of the measurement region (in directions perpendicular to the longitudinal axis of the body) are non-zero and given by the statistical distribution of the body's location. For yarn, the width and height may e.g. be 3 - 4 mm. Advantageously, the measurement region corresponds to the region where the elongate body is, in operation, at least in 90% of the time.

Measurement region 6 forms part of a slit 10, whose design is described in the next section.

Body 8 extends along a propagation direction X and, during the measurement, is e.g. continuously displaced along this propagation direction.X.

### Slit Design

Slit 10 is open at a first side 14a and a second side 14b (Fig. 2), with both these sides being transversally to, in particular perpendicular to, propagation direction X. Further, slit 10 is open at a third side 14c (Fig. 1), which extends along, in particular parallel to, propagation direction X.

Further, slit 10 has a bottom wall 16a and two lateral walls 16b, 16c, with bottom wall 16a connecting the two lateral walls 16b, 16c. All these walls extend along propagation direction X, in particular parallel thereto.

Prior to a measurement, body 8 can be inserted into measurement region 6 by inserting it from third side 14c into slit 10.

The device comprises an integral body 18, which forms bottom wall 16a as well as at least part of each lateral wall 16b, 16c.

Further, the device comprises a housing 20, which forms another part of each lateral wall 16b, 16c.

Housing 20 e.g. encloses all or at least part of the device.

A seam 22 is formed at the lateral walls 16b, 16c of slit 10 where a top edge 24 of integral body 18 meets a bottom edge 26 of housing 20.

Slit 10 can be viewed as having a bottom section 10a and a top section 10b. Bottom section 10a is adjacent to bottom wall 16 and extends up to edge 24 and is therefore laterally bordered by integral body 18. Top section 10b extends upwards from edge 24. In the embodiment shown, it is bordered by housing 20.

Measurement region 6 is located completely in bottom section 10a since elongate body 8 is advantageously kept away from seam 22 and edge 24 for the reasons mentioned above.

At the location of seam 22, the lateral walls 16b, 16c are smooth as defined above to reduce the risk of snagging elongate body 8 or fibers.

To ease the insertion of elongate body 8, the lateral walls 16b, 16c are flared outwards, i.e. the slit widens towards its top opening along the depth direction Y away from bottom wall 16a. In particular, the lateral walls 16a, 16b may be flared outwards at the location of seam 22.

Since bottom wall 16a and the lower parts (i.e. the parts in lower section 10a) of the lateral walls 16b, 16c are formed by a surface of integral body 18, all walls surrounding measurement region 6 can be manufactured without any seam or other obstacle that may snag a fiber.

Integral body 18 forms windows 28 in the lateral walls 16b, 16c adjacent to measurement region 6. These windows are transparent for the light from light source 2 and detectable by light detector 4.

Advantageously, integral body 18 is transparent. It may be used to guide the light as e.g. shown in Fig. 1.

As shown in Fig. 1, slit 10 is fairly deep for the reasons mentioned above. In particular, the distance D, measured along depth direction Y, from the center 30 of measurement region 6 to the upper edge 24 of integral body 18, is at least 13 mm, in particular at least 14 mm, in particular at least 18 mm.

To ensure that the slot remains easy to clean, depth D should, on the other hand, not be too large. Advantageously, D is smaller than 30 mm.

As illustrated in Fig. 1, if a fiber 32 is snagged at seam 22 or edge 24, it is typically short enough to not reach into measurement region 6, in particular because it is typically directed along propagation direction X.

To reduce the amount of possibly disturbing environmental light entering into measurement region 6, the width W of slit 10 in the direction Z perpendicular to propagation direction X and depth direction Y at the location of center 30 of measuring region 6 is advantageously at least 2.5 times, in particular at least 4 times, smaller than the distance D.

In particular, width W may be smaller than 5 mm.

Integral body 18 is, as mentioned, a single piece. Advantageously, the method for manufacturing the device comprises the step of casting the integral body 8, optionally with subsequent machining and/or coating steps.

Integral body 18 is advantageously of a single material, i.e. a single-component body, at least where it faces slit 10. Alternatively, it can be of two or more materials, e.g. a two-component body, which may e.g. be advantageously cast in a two-component casting process.

To ease the accurate alignment of integral body 18 in respect to housing 20 at the location of seam 22, the top edge of integral body 18 and the bottom edge of housing 20 at seam 22 may comprise projections 34 and recesses 36 cooperating to form an interlocking connection mutually positioning the two edges 24, 26 in direction Z perpendicular to directions X and Y. Also, these projections 34 and recesses 36 form a labyrinth reducing the risk of contaminants entering the device.

As mentioned, center 30 of measurement region 6 is defined by the time-average of the elongate body's position during the measurement. Typically, this position is defined by one or more guides 38, which may e.g. be placed adjacent to measurement slit 10, such as shown in Fig. 2. These guide(s) 38 is/are adapted to maintain elongate body 8 at the center 30 during the measurement.

### Notes

In the example above, elongate body 8 is a yarn. It must be noted, though, that it may also be another textile object, such as a yarn precursor, a textile ribbon, or a fiber.

In the embodiment above, the top opening of slit 10 is formed by housing 20. Alternatively, the top opening of slit 10 may also be formed by integral body 18.

Typically, housing 20 is of a material different from integral body 18. In particular, housing 20 may be of a non-transparent material, e.g. metal, thus preventing environmental light from entering the measuring region and/or integral body 18.

While there are shown and described presently preferred embodiments of the invention, it is to be distinctly understood that the invention is not limited thereto but may be otherwise variously embodied and practiced within the scope of the following claims.

## Claims

1. A sensor device for sensing at least one quality parameter of an elongate textile body comprising
a housing (20),
a measurement region (6) for the elongate textile body,
at least one light source (2) emitting light into said measurement region (6) and at least one light detector (4) receiving light from said measurement region (6),
a measurement slit (10) extending along a propagation direction (X) wherein
- said slit (10) is open at a first and second side (14a, 14b) transversal to said propagation direction (X), open at a third side (14c) along said propagation direction (X), and having two lateral walls (16b, 16c) and a bottom wall (16a) along said propagation direction (X), wherein said third side (14c) is opposite to said bottom wall (16a), and wherein a direction (Y) perpendicular to said propagation direction (X) and connecting said bottom wall (16a) and said third side (14c) defines a depth direction (Y), and
- said measurement region (6) is located in said slit (10),
an integral body (18) having a slit-facing surface forming at least part of each lateral wall (16b, 16c) and said bottom wall (16a),
wherein a distance (D), measured along said depth direction (Y), between a center (30) of said measurement region (6) and an edge (24) of said integral body (18) at said lateral walls (16b, 16c) is at least 13 mm.

2. The sensor device of claim 1 wherein said housing (20) forms part of each lateral wall (16b, 16c).

3. The sensor device of claim 2 wherein said housing (20) and said edge meet at a seam (22).

4. The sensor device of claim 3 wherein, at said seam (22), said lateral walls (16b, 16c) are smooth.

5. The sensor device of any of the claims 3 or 4 wherein, at said seam (22), said lateral walls (16b, 16c) are flared outwards.

6. The sensor device of any of the claims 3 to 5, wherein, at said seam (22), the edge (24) of said integral body (18) and an edge (26) of said housing (20) comprise projections (34) and recesses (36) cooperating to form an interlocking connection mutually positioning the edges (24, 26) in a direction (Z) perpendicular to said depth direction (Y) and said propagation direction (X) .

7. The sensor of any of the preceding claims wherein said integral body (18) forms windows (28) in said lateral walls (16b, 16c) adjacent to said measurement region (6), wherein said windows (28) are transparent for light from said light source (2) and detectable by said light detector (4).

8. The sensor device of any of the preceding claims wherein a width (W) of said slit (10) at the center (30) of said measurement region (6) in a direction (Z) perpendicular to said propagation direction (X) and said depth direction (Y) is at least 2.5 times, in particular at least 4 times smaller than said distance (D).

9. The sensor device of any of the preceding claims wherein said depth (D) is
at least 14 mm, in particular at least 18 mm, and/or
smaller 30 mm.

10. The sensor device of any of the preceding claims comprising one or more guides (38) adapted to maintain said elongate body at said center (30) of said measurement region (6).

11. A method for manufacturing the sensor device of any of the preceding claims comprising the step of casting said integral body (18).
